# EUROPEAN PATENT APPLICATION

(11) **EP 4 745 978 A1**
(43) Date of publication of application: **20.05.2026**
(21) Application number: 25216459.5
(22) Date of filing: 17.11.2025
(51) Int. Cl.: G16H 20/00, G06N 3/02, G06N 5/01, G06N 5/025, G06N 5/04, G06N 20/20, G16H 50/20, G16H 50/70

(54) **INFERENCE METHOD, INFERENCE APPARTUS, AND STORAGE MEDIUM**

(30) Priority: 18.11.2024 JP 2024201082
(71) Applicant: Canon Medical Systems Corporation, Otawara-shi, Tochigi 324-0036 (JP)
(72) Inventor: KANO, Yusuke, Otawara-shi, 324-0036 (JP)
(74) Representative: Marks & Clerk LLP

(57) **Abstract**

An inference method according to an embodiment includes acquiring attribute information for a target patient to be subjected to inference, and estimating a causal relationship between the attribute information for the target patient and a treatment effect for the target patient using a causal inference model including a decision tree model optimized based on knowledge information defining rules for determining a treatment to be applied to other patient and clinical information indicating relationship between attribute information for the other patient and other treatment effect observed after the treatment.

## Description

### FIELD

Embodiments described herein relate generally to an inference method, an inference apparatus, and a storage medium.

### BACKGROUND

In recent years, causal inference has been used to estimate causal effects in various fields including the medical field. For example, in the medical field, there is an interest in "individualized treatment effect (ITE) estimation" for estimating a treatment effect for each individual from attribute information for the patient through causal inference using machine learning.

### SUMMARY OF INVENTION

An inference method provided in an aspect of the present embodiment includes acquiring attribute information for a target patient to be subjected to inference; and estimating a causal relationship between the attribute information for the target patient and a treatment effect for the target patient using a causal inference model including a decision tree model optimized based on knowledge information defining rules for determining a treatment to be applied to other patient and clinical information indicating relationship between attribute information for the other patient and other treatment effect observed after the treatment.

The knowledge information may include first knowledge information for specifying, for each treatment type, a condition of other patient for whom the treatment is recommended or a condition of other patient for whom the treatment is not recommended.

The knowledge information may include second knowledge information for specifying, for each attribute of other patient, a treatment type in which the attribute is advantageous or a treatment type in which the attribute is disadvantageous.

At least some parameters of the decision tree model may be determined so as to match the rules for determining the treatment in the knowledge information.

The causal inference model may be optimized by giving a penalty when a contradiction occurs in comparison between an estimation result and a treatment determination based on the knowledge information.

The causal inference model may be optimized based on an estimated value obtained by a machine learning model that estimates a treatment effect for other patient.

The decision tree model may include a first parameter corresponding to a branching condition of an internal node for allocating the target patient to one leaf node of the decision tree, and a second parameter corresponding to a condition for converting information on the leaf node into an estimated value.

The second parameter may be a parameter that converts a representative feature representation vector of a patient population consisting of one or more other patients allocated to a leaf node into a representative value of the estimated value in the patient population.

The causal inference model may further include a neural network that extracts the feature representation vector that does not depend on treatment allocation based on the attribute information for the other patient.

The causal inference model may be an ensemble learning model using the decision tree model as a weak learner.

The parameters of the causal inference model may be updated as the knowledge information is updated.

The causal inference model may further include a neural network that estimates an individualized treatment effect based on a result of estimation by the decision tree model and the attribute information for the other patient.

An inference apparatus provided in an aspect of the present embodiment includes an acquisition unit configured to acquire attribute information for a target patient to be subjected to inference; and an estimation unit configured to estimate a causal relationship between the attribute information for the target patient and a treatment effect for the target patient using a causal inference model including a decision tree model optimized based on knowledge information defining rules for determining a treatment to be applied to other patient and clinical information indicating relationship between attribute information for the other patient and other treatment effect observed after the treatment.

A storage medium provided in an aspect of the present embodiment that non-transiently stores a program for causing a computer to execute acquiring attribute information for a target patient to be subjected to inference; and estimating a causal relationship between the attribute information for the target patient and a treatment effect for the target patient using a causal inference model including a decision tree model optimized based on knowledge information defining rules for determining a treatment to be applied to other patient and clinical information indicating relationship between attribute information for the other patient and other treatment effect observed after the treatment.

### BRIEF DESCRIPTION OF THE DRAWINGS

FIG. 1 is a block diagram illustrating an example of a configuration of an inference apparatus according to a first embodiment;
FIG. 2 is a flowchart illustrating an example of a procedure of a causal inference model construction process executed by the inference apparatus according to the first embodiment;
FIG. 3 is a diagram illustrating an example of learning data according to the first embodiment;
FIG. 4 is a diagram for explaining representation learning according to the first embodiment;
FIG. 5 is a diagram for explaining an example in which a decision tree model is learned according to the first embodiment;
FIG. 6 is a diagram for explaining an example in which a decision tree model is learned according to the first embodiment;
FIG. 7 is a diagram for explaining an example in which a neural network is learned according to the first embodiment;
FIG. 8 is a flowchart illustrating an example of a procedure of an inference process executed by the inference apparatus according to the first embodiment;
FIG. 9 is a flowchart illustrating an example of a procedure of a causal inference model construction process executed by an inference apparatus according to a second embodiment; and
FIG. 10 is a diagram for explaining construction of a causal inference model according to the second embodiment.

### DETAILED DESCRIPTION

Hereinafter, embodiments of an inference method, an inference apparatus, and a storage medium will be described in detail with reference to the drawings. Note that the inference method, the inference apparatus, and the storage medium according to the present application are not limited by the following embodiments.

### First Embodiment

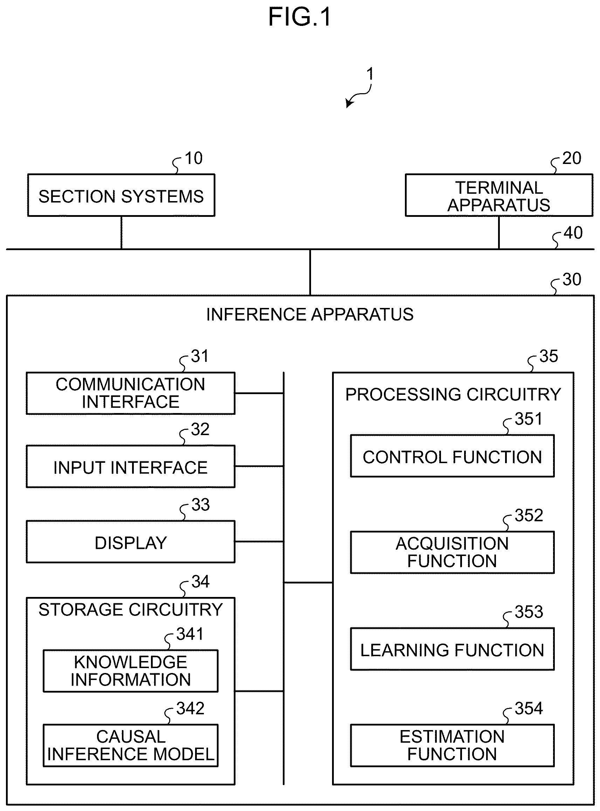

Hereinafter, a configuration of an inference apparatus according to a first embodiment will be described with reference to FIG. 1. FIG. 1 is a block diagram illustrating an example of the configuration of the inference apparatus according to the first embodiment. For example, an inference apparatus 30 is included in a medical information processing system 1 including section systems 10 and a terminal apparatus 20. The section systems 10, the terminal apparatus 20, and the inference apparatus 30 are communicably connected to each other via a network 40. Here, the network includes, for example, an in-hospital local area network (LAN) installed in a hospital or a wide area network (WAN).

Note that various other apparatuses and systems such as medical image diagnostic apparatuses may be connected to the network illustrated in FIG. 1. The medical image diagnostic apparatuses are apparatuses that capture an image of a subject to generate a medical image, and include, for example, an X-ray computed tomography (CT) apparatus, a magnetic resonance imaging (MRI) apparatus, an X-ray diagnostic apparatus, an ultrasonic diagnostic apparatus, a single photon emission computed tomography (SPECT) apparatus, a positron emission computed tomography (PET) apparatus, and the like.

The section systems 10 includes various systems such as a hospital information system (HIS), a radiology information system (RIS), a picture archiving and communication system (PACS), a diagnosis report system, a laboratory information system (LIS), a rehabilitation department system, a dialysis department system, and a surgery department system. The section systems 10 manages subject information (attribute information for a patient) for each subject (patient), and transmits the subject information in response to requests from the terminal apparatus 20 and the inference apparatus 30. Here, the subject information (attribute information for the patient) includes, for example, various medical images collected by the medical image diagnostic apparatus, measurement values obtained using the medical images, gender, age, various measured values (such as height, weight, and blood pressure), whether medication is taken, etc., and is managed in association with information regarding subject information data (such as collection date and time and data storage location).

The terminal apparatus 20 is an apparatus operated by a doctor working in a hospital or the like. For example, the terminal apparatus 20 is realized by a personal computer, a tablet PC, a PDA, a mobile phone such as a smartphone, or the like. The terminal apparatus 20 displays various types of information on its own display and receives various operations via its own input interface. Here, the terminal apparatus 20 can transmit a processing request to the inference apparatus 30 in response to an operation of a doctor or the like, and receive a processing result from the inference apparatus 30.

As illustrated in FIG. 1, the inference apparatus 30 includes a communication interface 31, an input interface 32, a display 33, storage circuitry 34, and processing circuitry 35. For example, the inference apparatus 30 is realized by a computer apparatus such as a server or a workstation.

The communication interface 31 controls transfer and communication of various types of data transmitted and received between the inference apparatus 30 and each apparatus connected via the network 40. Specifically, the communication interface 31 is connected to the processing circuitry 35, and transmits data received from each apparatus on the network 40 to the processing circuitry 35 or transmits data received from the processing circuitry 35 to each apparatus on the network 40. For example, the communication interface 31 is realized by a network card, a network adapter, a network interface controller (NIC), or the like.

The input interface 32 receives input operations for various instructions and various types of information from an operator. Specifically, the input interface 32 is connected to the processing circuitry 35, converts an input operation received from an operator into an electric signal, and transmits the electric signal to the processing circuitry 35. For example, the input interface 32 is realized by a trackball, a switch button, a mouse, a keyboard, a touch pad through which an input operation is performed by touching an operation surface, a touch screen in which a display screen and a touch pad are integrated, a non-contact input interface using an optical sensor, a voice input interface, or the like. Note that, in the present specification, the input interface 32 is not limited to one including physical operation components such as a mouse and a keyboard. For example, examples of the input interface 32 also include an electric signal processing circuit that receives an electric signal corresponding to an input operation from an external input device provided separately from the device and transmits the electric signal to a control circuit.

The display 33 displays various types of information and various types of data. Specifically, the display 33 is connected to the processing circuitry 35, and displays various types of information and various types of data received from the processing circuitry 35. For example, the display 33 is realized by a liquid crystal display, a cathode ray tube (CRT) display, a touch panel, or the like.

The storage circuitry 34 stores various types of data and various programs. Specifically, the storage circuitry 34 is connected to the processing circuitry 35, and stores data received from the processing circuitry 35, or reads data stored therein and transmits the data to the processing circuitry 35. For example, the storage circuitry 34 is realized by a semiconductor memory element such as a read only memory (ROM), a random access memory (RAM), or a flash memory, a hard disk, an optical disk, or the like. For example, the storage circuitry 34 stores subject information and various programs received from the section systems 10. Furthermore, as illustrated in FIG. 1, the storage circuitry 34 stores knowledge information 341 and a causal inference model 342. The knowledge information 341 is various guidelines regarding medical care, and will be described in detail later. The causal inference model 342 is a model that estimates a treatment effect in response to an input of attribute information for a target patient, and will be described in detail later. Note that the storage circuitry 34 may be realized by a cloud computer connected to the inference apparatus 30 via the network 40.

The processing circuitry 35 controls the entire inference apparatus 30. Specifically, the processing circuitry 35 controls transmission and reception of information between the apparatuses on the network 40, and controls various processes regarding the learning of the causal inference model 342 and inference using the causal inference model 342. Note that the processing circuitry 35 can also perform various processes in response to input operations via the input interface 32.

The example of the configuration of the inference apparatus 30 according to the present embodiment has been described above. For example, the inference apparatus 30 is installed in a medical facility such as a hospital, and assists a user such as a doctor in estimating a treatment effect.

The individualized treatment effect estimation using the attribute information for the patient can be performed individually for a wide range of target patients, but the use of individual data may lead to incorrect inference (there may be bias). In addition, in the individualized treatment effect estimation, neural networks are often used, and it is difficult to understand how they are constructed (low interpretability).

On the other hand, clinical guidelines have been conventionally used to support medical decision-making. Although clinical guidelines are less biased and highly reliable as they have been developed by randomized controlled trials, they are recommendations based on average results and have certain limitations in estimating individualized treatment effects. In addition, the clinical guidelines cannot be used by patients other than applicable patients because they are suitable for specific clinical situations.

Therefore, the inference apparatus 30 according to the present embodiment constructs a causal inference model in which treatment effect estimation using individual patient data and clinical guidelines are combined, thereby making it possible to accurately estimate a treatment effect for each patient. Specifically, the inference apparatus 30 constructs a causal inference model including a decision tree model optimized based on the clinical guidelines and clinical information indicating a relationship between the attribute information for the patient and a treatment effect observed after treatment, and estimates the treatment effect using the causal inference model. The causal inference model constructed in this way has the advantage of the individualized treatment effect estimation, which enables individual estimation for a wide range of patients, and the advantage of the clinical guidelines, which are less biased and highly reliable, thereby making it possible to accurately estimate a treatment effect for each patient. Hereinafter, the inference apparatus 30 having such a configuration will be described in detail.

For example, as illustrated in FIG. 1, the processing circuitry 35 of the inference apparatus 30 executes a control function 351, an acquisition function 352, a learning function 353, and an estimation function 354. Here, the acquisition function 352 is an example of an acquisition unit. Furthermore, the estimation function 354 is an example of an estimation unit.

The control function 351 controls various processes based on various requests input from the operator via the input interface 32 and various programs and various types of data read from the storage circuitry 34. For example, the control function 351 causes the display 33 to display a graphical user interface (GUI) for receiving an input related to the construction of the causal inference model 342, an input related to the estimation of the treatment effect by the causal inference model 342, or the like, an estimation result, etc.

The acquisition function 352 acquires various types of information regarding patients. Specifically, the acquisition function 352 acquires learning data (clinical information for a plurality of other patients) used for learning the causal inference model 342 and attribute information for a target patient to be subjected to inference by the causal inference model 342. For example, the acquisition function 352 acquires the learning data or the attribute information for the target patient from the section systems 10 in response to an input operation input via the input interface 32.

The learning function 353 constructs the causal inference model 342 and stores the causal inference model 342 in the storage circuitry 34. Specifically, the learning function 353 generates a causal inference model including a decision tree model optimized based on knowledge information defining rules for determining a treatment to be applied to other patient and clinical information indicating relationship between attribute information for the other patient and other treatment effect observed after the treatment, and stores the causal inference model in the storage circuitry 34. Note that the process executed by the learning function 353 will be described in detail later.

The estimation function 354 performs treatment effect estimation using the causal inference model 342. Specifically, the estimation function 354 estimates the treatment effect of the target patient by inputting the attribute information for the target patient acquired by the acquisition function 352 into the causal inference model 342 constructed by the learning function 353. That is, the estimation function 354 estimates a causal relationship between the attribute information for the target patient and the treatment effect for the target patient using the causal inference model 342 including the decision tree model optimized based on the knowledge information defining the rules for determining a treatment to be applied to other patient and the clinical information indicating the relationship between the attribute information for the other patient and other treatment effect observed after the treatment. Note that the process executed by the estimation function 354 will be described in detail later.

The processing circuitry 35 described above is realized by, for example, a processor. In that case, each of the processing functions described above is stored in the storage circuitry 34 in the form of a program that can be executed by a computer. Then, the processing circuitry 35 reads and executes each program stored in the storage circuitry 34, thereby realizing a function corresponding to each program. In other words, the processing circuitry 35 has each processing function illustrated in FIG. 1 in a state where each program is read.

Note that the processing circuitry 35 may be configured by combining a plurality of independent processors, and each of the processors may execute a program, thereby realizing each processing function. Furthermore, each processing function of the processing circuitry 35 may be realized by being appropriately distributed or integrated in one or more processing circuits. In addition, each processing function of the processing circuitry 35 may be realized by combining hardware such as a circuit and software. Furthermore, here, an example in which the programs corresponding to the respective processing functions are stored in the single storage circuitry 34 has been described, but the embodiment is not limited thereto. For example, the programs corresponding to the respective processing functions may be stored in a plurality of storage circuits in a distributed manner, and the processing circuitry 35 may read and execute each program from each storage circuit. Note that some of the processing functions of the processing circuitry 35 may be realized by a cloud computer connected to the inference apparatus 30 via the network 40.

Next, a procedure of the process executed by the inference apparatus 30 according to the first embodiment will be described. Here, the inference apparatus 30 performs a process of constructing a causal inference model 342 and a process of estimation using the causal inference model 342. Hereinafter, these processes will be described in order.

### Construction of Causal Inference Model

As described above, the inference apparatus 30 constructs a causal inference model for individualized treatment effect estimation using a decision tree model. The decision tree model is easy to understand as to how inference was made (high interpretability), and its structure makes it easy to apply clinical guidelines. Here, various approaches can be considered for learning a decision tree model in consideration of causality and knowledge. For example, a causal tree is known as an existing technique in which a general decision tree such as a "classification and regression tree (CART)" is applied to a causal inference task.

However, in the learning method based on the greedy method such as "CART", an optimal branch is determined only at each individual node in the decision tree, and thus the branch may not be optimal when viewed from the whole decision tree. Therefore, in the present embodiment, a method is used in which branching conditions at a plurality of nodes in a decision tree are simultaneously optimized. As such a method, there is "GradTree", which learns a decision tree model using a gradient descent method, but it cannot be directly applied to a causal inference task. For this reason, in the present embodiment, "GradTree" is applied to a causal inference task using representation learning in causal inference. Note that, to learn a decision tree model using "GradTree", the method described in the literature "Sascha Marton, et al. "GradTree: Learning Axis-Aligned Decision Trees with Gradient Descent" arXiv:2305.03515v7" can be used.

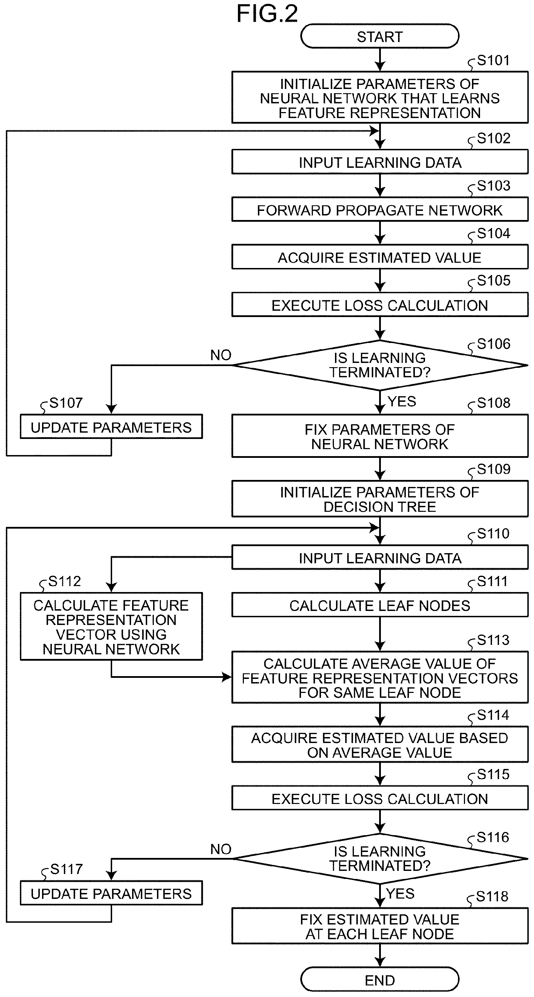

FIG. 2 is a flowchart illustrating an example of a procedure of a causal inference model construction process executed by the inference apparatus 30 according to the first embodiment. Here, FIG. 2 illustrates a case where representation learning using a neural network is performed in steps S101 to S108, and a decision tree model learned in steps S109 to S118. Note that the processing of each step illustrated in FIG. 2 is realized by the processing circuitry 35 reading a program corresponding to the learning function 353 from the storage circuitry 34 and executing the program.

For example, as illustrated in FIG. 2, in the inference apparatus 30 according to the present embodiment, the learning function 353 initializes parameters of the neural network that learns feature representation (step S101), inputs learning data acquired by the acquisition function 352 (step S102), forward propagates the network (step S103), and acquires an estimated value (step S104).

Subsequently, the learning function 353 calculates a loss related to the acquired estimated value (step S105), and determines whether to terminate the representation learning (whether the loss satisfies the condition) (step S106).
Here, when the representation learning is not terminated (step S106, No), the learning function 353 updates the parameters of the neural network (step S107) and inputs learning data again (step S102).

On the other hand, when the representation learning is terminated (step S106, Yes), the learning function 353 fixes the parameters of the neural network (step S108) and initializes parameters of the decision tree model (step S109). Subsequently, the learning function 353 inputs learning data corresponding to attribute information for a plurality of other patients (step S110), thereby calculating leaf nodes of the decision tree model (step S111).

Here, the learning function 353 inputs the learning data input into the decision tree model into the neural network learned in steps S101 to S108, thereby calculating a feature representation vector corresponding to each patient (step S112). Further, the learning function 353 calculates an average value of the feature representation vectors of the patients classified into the same leaf node of the decision tree model (step S113), and acquires an estimated value corresponding to each average value (step S114).

Subsequently, the learning function 353 calculates a loss related to the acquired estimated value (step S115), and determines whether to terminate the learning of the decision tree model (step S116). Here, when the learning of the decision tree model is not terminated (step S116, No), the learning function 353 updates the parameters of the decision tree model (step S117) and inputs learning data again (step S110).

On the other hand, when the learning of the decision tree model is terminated (step S116, Yes), the learning function 353 fixes the estimated value at each leaf node to the average value at the time of the end of the learning (step S118), and terminates the process.

Hereinafter, the causal inference model construction process executed by the inference apparatus 30 will be described in detail.

### Learning Data

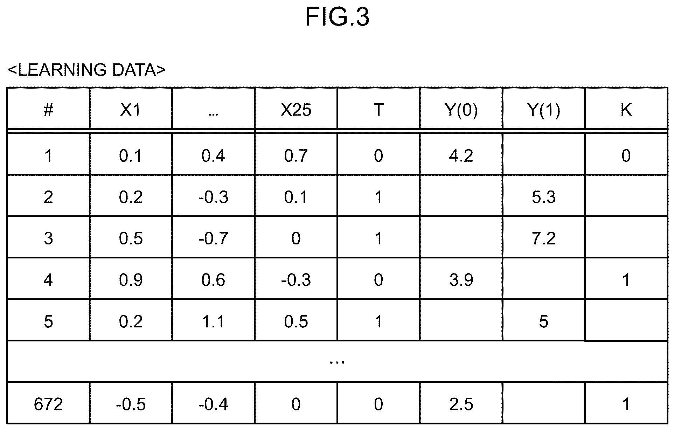

The learning data used in the construction of the causal inference model 342 includes clinical information indicating relationship between attribute information for other patient and other treatment effect observed after the treatment. Specifically, the learning data includes attribute information, treatment effects, treatment contents, knowledge labels, etc. FIG. 3 is a diagram illustrating an example of the learning data according to the first embodiment. Here, in FIG. 3, each row represents other patient. For example, as illustrated in FIG. 3, the learning data includes, for each of other patients, attribute information denoted by "X1 to X25", a treatment content denoted by "T", treatment effects denoted by "Y(0)" and "Y(1)", and a knowledge label denoted by "K".

Here, as illustrated in FIG. 3, the attribute information is converted into a numerical value for each piece of information. In addition, the treatment effect is converted into a larger numerical value as the treatment effect is better. For example, in the example illustrated in FIG. 3, the treatment effect "Y(0)" when a treatment denoted by "0" is performed for disease "Y" and the treatment effect "Y(1)" when a treatment denoted by "1" is performed for disease "Y" are illustrated, and the treatment effect of the treatment actually performed on each of other patients is indicated by a numerical value. Furthermore, the knowledge label "K" refers to a recommended treatment, and is set in advance based on the clinical guidelines.

For example, for other patient "#1", the knowledge level "K" is "0" (that is, the recommended treatment is a treatment denoted by "0"), the actually performed treatment is "treatment content (T):0", and the treatment effect "Y(0)" is "4.2". The learning data includes a plurality of pieces of clinical information for such other patients. In the learning data, the treatment effects of the treatments that have not actually been performed and the knowledge levels of the other patients for whom the recommended treatments are not defined in the clinical guidelines are blank.

### Representation Learning

In the representation learning described in steps S101 to S108 of FIG. 2, the learning function 353 learns feature representation for learning a structure of a decision tree model. Specifically, the learning function 353 performs representation learning using a neural network so as to obtain feature representation vectors useful in learning a decision tree model. That is, the causal inference model constructed by the learning function 353 further includes a neural network that extracts the feature representation vector that does not depend on treatment allocation based on the attribute information for the other patient.

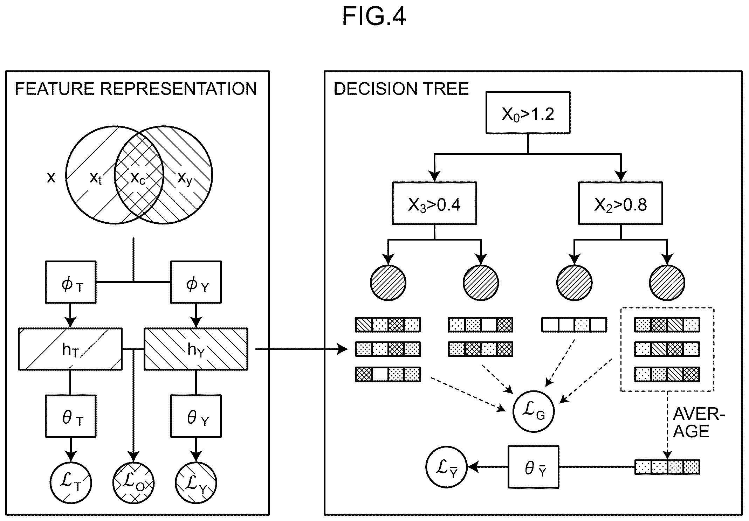

FIG. 4 is a diagram for explaining the representation learning according to the first embodiment. As illustrated in FIG. 4, in the representation learning (feature representation in the drawing), a parameter "ϕ" for extracting a feature representation vector "h" used to learn the structure of the decision tree model in the process of constructing the causal inference model 342 and a parameter "θ" for converting the feature representation vector into an estimated value are optimized. For example, in a case where a causal inference model for estimating a treatment effect for disease "Y" is constructed, a parameter "ϕ_{Y}" for extracting a feature representation vector "h_{Y}" and a parameter "θ_{Y}" for converting the feature representation vector "h_{Y}" into an estimated value "L_{Y}" of treatment effect are optimized.

Here, in the representation learning, learning is performed so as to obtain a feature representation vector that is less biased in inference in the decision tree model. That is, learning is performed such that a feature representation vector that is less biased is obtained when the feature representation vector is converted into an estimated value. As a result, at each leaf node of the decision tree model, the relationship between the feature representation vector and the estimated value is linked by a common, unbiased relationship.

For example, the learning function 353 obtains a feature representation vector that is less biased by removing biased information and reducing data imbalance in the learning data "X" input into the neural network. Furthermore, the learning function 353 optimizes the parameter "ϕ_{Y}" and the parameter "θ_{Y}" based on loss calculation using the estimated value "L_{Y}" and the actual treatment effect included in the clinical information. Here, the estimated value "L_{Y}" may be any of an estimated value of treatment effect in a case where the treatment content is "0", an estimated value of treatment effect in a case where the treatment content is "1", and a difference between the estimated value of treatment effect in a case where the treatment content is "0" and the estimated value of treatment effect in a case where the treatment content is "1".

### Learning of Decision Tree Model

In the learning of the decision tree model described in steps S109 to S118 of FIG. 2, the learning function 353 learns a decision tree model using the neural network learned by the representation learning. For example, the learning function 353 learns a decision tree model that estimates a treatment effect for disease "Y" using the neural network in which the parameter "ϕ_{Y}" and the parameter "θ_{Y}" are optimized. As an example, as illustrated in FIG. 4, the learning function 353 optimizes parameters (such as X₀, X₂, and X₃ or 1.2, 0.4, and 0.8 in the drawing) corresponding to branching conditions (such as features to be branched and thresholds) of internal nodes in the decision tree model by using an average value of feature representation vectors at the leaf node of the decision tree model. Here, the parameter to be optimized corresponds to a first parameter corresponding to the branching condition of the internal node for allocating the target patient to one of the leaf nodes of the decision tree. In addition, the parameter "θ" optimized by the representation learning corresponds to a second parameter corresponding to the condition for converting information on the leaf node into an estimated value. Specifically, the second parameter is a parameter that converts a representative feature representation vector of a patient population consisting of one or more other patients allocated to the leaf node into a representative value of the estimated value in the patient population.

Here, the learning function 353 can use the knowledge information 341 in the optimization of the parameter corresponding to the branching condition of the internal node of the decision tree model. The knowledge information 341 defines rules for determining a treatment to be applied to other patient, and includes first knowledge information for specifying, for each treatment type, a condition of other patient for whom the treatment is recommended or a condition of other patient for whom the treatment is not recommended, and second knowledge information for specifying, for each patient attribute, a treatment type in which the attribute is advantageous or a treatment type in which the attribute is disadvantageous.

The first knowledge information is information on a combination of a condition and recommendation/non-recommendation, and is, for example, recommendation knowledge such as "treatment type A is recommended for age ≥ 60 years" or non-recommendation knowledge such as "treatment type B is contraindicated for age ≥ 60 years". That is, the first knowledge information is information that can be used alone to determine a recommended treatment (or a non-recommended treatment) for a specific patient.

Note that the condition in the first knowledge information is not limited to a simple condition such as age, and an index value derived based on a plurality of attributes may be used. For example, a condition such as "if European System for Cardiac Operative Risk Evaluation II (Euro Score II)", which is a model for estimating a mortality rate of cardiac surgery, is "equal to or more than..." may be used. That is, the first knowledge information may be conditioned on an output of a model constructed by AI.

The second knowledge information is information obtained by subgroup analysis, and is, for example, knowledge such as "treatment type A is more effective in a patient group aged 60 years or older than in a patient group aged under 60 years". In this example, even if the target patient is 60 years old or older, treatment type A is not necessarily the optimal treatment depending on the other attributes. That is, since the second knowledge information does not specify the condition of the patient, a recommended treatment (or a non-recommended treatment) cannot be determined for a specific patient by the information alone. Note that, similarly to the first knowledge information, the condition in the second knowledge information is not limited to a simple condition such as age, and an index value derived based on a plurality of attributes may be used.

The learning function 353 learns a decision tree model by integrating the knowledge information 341 described above and causality. Specifically, the learning function 353 integrates the knowledge information into the learning of the decision tree model by at least one of fixing some of the determination rules at the internal nodes of the decision tree model to be learned using the determination rules of the knowledge information 341 and using the knowledge information 341 in the loss calculation for the output of the decision tree model.

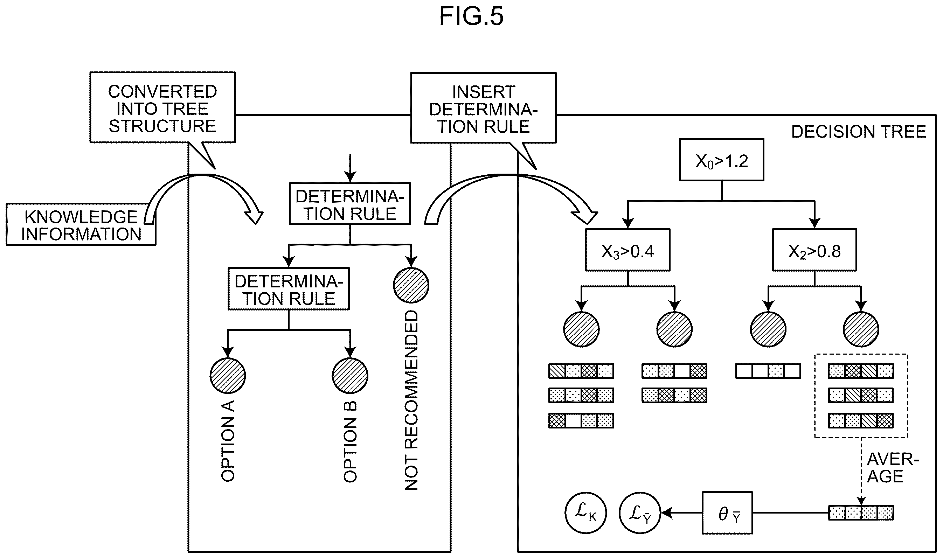

For example, the learning function 353 performs learning such that at least some of the parameters of the decision tree model match rules for determining a treatment in the knowledge information 341. FIG. 5 is a diagram for explaining an example in which a decision tree model is learned according to the first embodiment. For example, as illustrated in FIG. 5, the learning function 353 converts knowledge information into a tree structure with determination rules (conditions) of the knowledge information as branching conditions, and performs learning so as to insert the tree structure into the decision tree in learning the decision tree model. Note that the position at which the tree structure is inserted in the decision tree model is arbitrary, and parameter optimization is executed while changing the insertion position.

Furthermore, for example, the learning function 353 optimizes the decision tree model by giving a penalty when a contradiction occurs in the comparison between the estimation result and the treatment determination based on the knowledge information. The learning function 353 classifies other patients into each leaf node, by inputting learning data into the decision tree model, in learning the decision tree model using the learning data. Here, the learning function 353 acquires a feature representation vector (schematically indicated by four rectangles below the leaf node of the decision tree in FIG. 5) for each of the other patients, by inputting attribute information for other patients into the neural network learned in the representation learning.

The learning function 353 calculates an average value obtained by averaging the feature representation vectors of the respective other patients at each leaf node. For example, the learning function 353 calculates an average value obtained by averaging feature representation vectors of three other patients classified into the right-end leaf node in FIG. 5.

The learning function 353 executes loss calculation by converting the average value of the feature representation vectors calculated at each leaf node into an estimated value by the parameter "θ" learned by the representation learning. Here, the learning function 353 can integrate the knowledge information into the decision tree model by performing learning so as to give a penalty in a case where a contradiction occurs between the estimated value and the treatment determination based on the knowledge information. For example, in the first knowledge information, since recommendation/non-recommendation can be determined according to the condition, it is possible to determine whether there is a contradiction by comparing a result of determining treatment for other patient classified as a leaf node based on the first knowledge information with an estimated value.

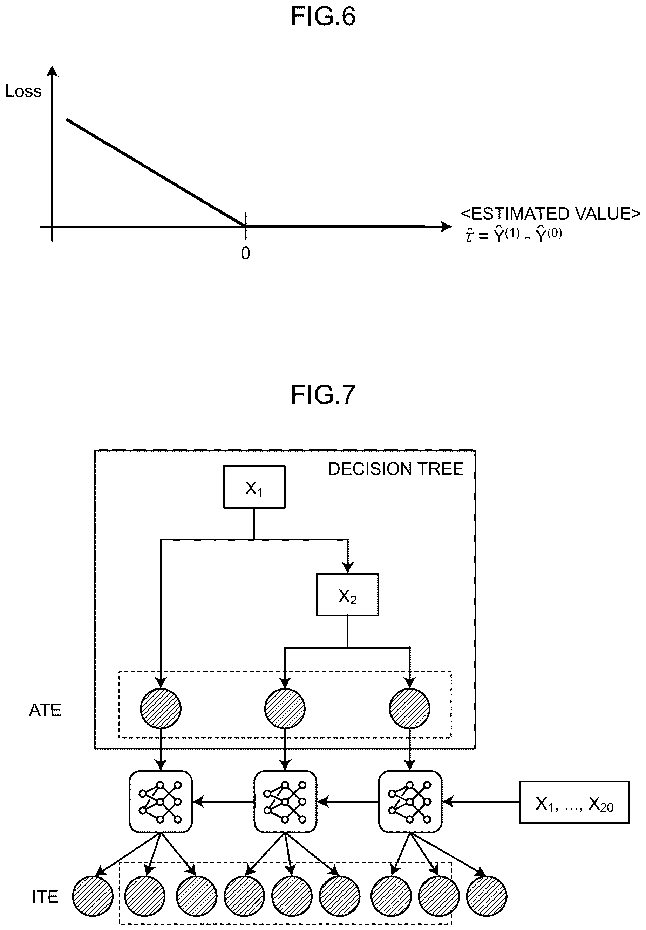

FIG. 6 is a diagram for explaining an example in which a decision tree model is learned according to the first embodiment. Here, FIG. 6 illustrates a loss in a case where treatment content "1" is recommended as a treatment of disease "Y" for other patient classified into a leaf node. For example, as illustrated in FIG. 6, the learning function 353 performs learning so as to give a loss in a case where the sign of the estimated value of treatment effect of treatment content "1" relative to treatment content "0" (the estimated value represented by the mathematical formula in the drawing) is negative. That is, the learning function 353 gives a loss in a case where the difference between the estimated value of treatment effect of treatment content "1" and the estimated value of treatment effect of treatment content "0" is negative.

Furthermore, in a case where the second knowledge information is integrated into the decision tree model, the learning function 353 extracts a plurality of other patients corresponding to the patient population defined in the second knowledge information from the learning data, determines whether there is a contradiction by comparing an average value of the extracted estimated values of treatment effects for the plurality of other patients with a result of determination based on the second knowledge information, and gives a loss.

In the learning of the causal inference by the decision tree model, the learning function 353 optimizes the parameter corresponding to the branching condition of the internal node of the decision tree model while inserting the tree structure based on the above-described knowledge information and performing the loss calculation using the knowledge information.

When the first parameter of the decision tree model is optimized by the above-described learning, the learning function 353 fixes the estimated value at each leaf node after the optimization (the estimated value converted from the average value of feature representation vectors of other patients classified into each leaf node) as the estimated value for each leaf node. The learning function 353 stores the decision tree model constructed as described above in the storage circuitry 34 as the causal inference model 342.

By using the causal inference model 342 including the decision tree model constructed as described above, individual estimation can be performed for a wide range of patients, an estimation result that is less biased and highly reliable can be obtained, thereby making it possible to accurately estimate a treatment effect for each patient.

Note that the learning function 353 can update the parameters of the decision tree model in accordance with the update of the knowledge information. For example, when the knowledge information 341 stored in the storage circuitry 34 is updated, the learning function 353 re-optimizes the parameters of the decision tree model based on the updated knowledge information 341.

### Learning of Neural Network for Estimating Individualized Treatment Effects

In the decision tree model described above, an estimated value converted from an average value of feature representation vectors of other patients classified into each leaf node is obtained. That is, the treatment effect estimated in the decision tree model corresponds to the average treatment effect for the population. The inference apparatus 30 according to the present embodiment can also construct the causal inference model 342 including a neural network that estimates a difference between the average treatment effect and the individualized treatment effect estimated by the decision tree model.

In this case, the learning function 353 constructs the causal inference model 342 further including a neural network that estimates an individualized treatment effect, for example, based on the result of estimation by the decision tree model and the attribute information for the other patient.

FIG. 7 is a diagram for explaining an example in which a neural network is learned according to the first embodiment. As illustrated in FIG. 7, the learning function 353 constructs a neural network that estimates an "individualized treatment effect (ITE)" by using the "average treatment effect (ATE)" estimated by the decision tree model and the clinical information for the other patient.

For example, for each leaf node of the decision tree model, the learning function 353 learns a neural network using the estimated value of the leaf node and the clinical information for the other patient classified into the leaf node. Here, the learning function 353 optimizes the parameters in the neural network by performing loss calculation using the estimated values estimated by the neural network and the actual treatment effects included in the clinical information for the other patients. The learning function 353 stores the causal inference model 342 including the neural network for each leaf node with the optimized parameters and the decision tree model in the storage circuitry 34.

### Inference by Causal Inference Model

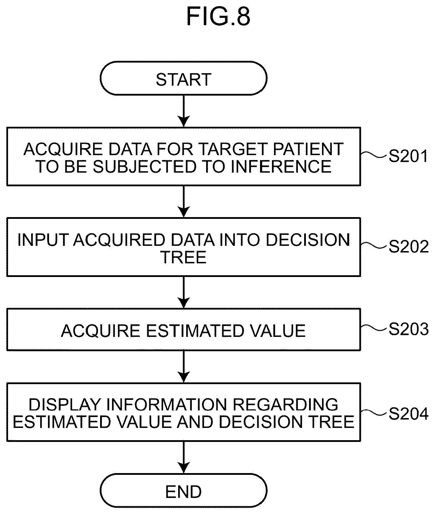

The estimation function 354 estimates a treatment effect for each patient using the causal inference model 342 stored by the storage circuitry 34. FIG. 8 is a flowchart illustrating an example of a procedure of an inference process executed by the inference apparatus 30 according to the first embodiment. Note that FIG. 8 illustrates a process in a case where a decision tree model is stored as the causal inference model 342.

As illustrated in FIG. 8, in the inference apparatus 30 according to the present embodiment, the acquisition function 352 acquires data (attribute information) for a target patient to be subjected to inference (step S201). Note that this processing is realized by the processing circuitry 35 reading a program corresponding to the acquisition function 352 from the storage circuitry 34 and executing the program.

Subsequently, the estimation function 354 inputs the acquired data for the target patient into the decision tree model (step S202), and acquires an estimated value (step S203). Note that this processing is realized by the processing circuitry 35 reading a program corresponding to the estimation function 354 from the storage circuitry 34 and executing the program.

Subsequently, the control function 351 causes the display 33 to display information regarding the estimated value and the decision tree (step S204). Note that this processing is realized by the processing circuitry 35 reading a program corresponding to the control function 351 from the storage circuitry 34 and executing the program.

Hereinafter, the inference process executed by the inference apparatus 30 will be described in detail.

### Inference Process

In the inference process described in steps S201 to S203 of FIG. 8, first, the acquisition function 352 acquires the attribute information for the target patient designated by an operator via the input interface 32. For example, the operator inputs information (such as patient name or patient ID) on a target patient who is not treated via the input interface 32. The acquisition function 352 acquires attribute information associated with the information input by the operator from the section systems 10.

The estimation function 354 acquires an estimated value by inputting the attribute information for the target patient acquired by the acquisition function 352 into the decision tree model. Specifically, the estimation function 354 acquires an estimated value (average treatment effect) associated with the leaf node into which the input target patient is classified.

### Display Process

As described in step S204 of FIG. 8, the control function 351 causes the display 33 to display the estimated value acquired by the estimation function 354 and the information regarding the decision tree model. For example, the control function 351 displays the estimated value (average treatment effect) acquired by the estimation function 354, and also displays display information showing the tree structure of the decision tree model and the determination rules (including the determination rules of the knowledge information) for each node. As a result, the operator can check the estimated treatment effect, and also can check how the estimated treatment effect is estimated.

Note that, in the above-described example, the causal inference model 342 is a decision tree model, but the causal inference model 342 may include a neural network that estimates ITE. In such a case, the estimation function 354 acquires a target patient-specific estimated value output from the neural network of the causal inference model 342 in response to an input of attribute information for a target patient. The control function 351 displays the target patient-specific estimated value acquired by the estimation function 354, and also displays display information showing the tree structure of the decision tree model and the determination rules (including the determination rules of the knowledge information) for each node.

As described above, according to the first embodiment, the inference apparatus 30 acquires attribute information for a target patient to be subjected to inference, and estimates a causal relationship between the attribute information for the target patient and a treatment effect for the target patient using a causal inference model including a decision tree model optimized based on knowledge information defining rules for determining a treatment to be applied to the other patient and clinical information indicating a relationship between the attribute information for the other patient and the other treatment effect observed after the treatment. Therefore, the inference apparatus 30 according to the first embodiment can perform individual estimation for a wide range of patients, and can obtain an estimation result that is less biased and highly reliable, thereby making it possible to accurately estimate a treatment effect for each patient. Furthermore, the inference apparatus 30 can perform estimation with high interpretability as to how the treatment effect is estimated.

Furthermore, according to the first embodiment, the knowledge information 341 includes first knowledge information for specifying, for each treatment type, a condition of other patient for whom the treatment is recommended or a condition of other patient for whom the treatment is not recommended. Furthermore, the knowledge information 341 includes second knowledge information for specifying, for each attribute of the other patient, a treatment type in which the attribute is advantageous or a treatment type in which the attribute is disadvantageous. Therefore, the inference apparatus 30 according to the first embodiment can integrate various clinical guidelines.

In addition, according to the first embodiment, at least some parameters of the decision tree model are determined so as to match the rules for determining the treatment in the knowledge information. Therefore, the inference apparatus 30 according to the first embodiment can appropriately incorporate clinical guidelines into the decision tree model.

Furthermore, according to the first embodiment, the causal inference model is optimized by giving a penalty when a contradiction occurs in comparison between an estimation result and a treatment determination based on the knowledge information 341. Therefore, the inference apparatus 30 according to the first embodiment can appropriately perform optimization using the clinical guidelines.

In addition, according to the first embodiment, the decision tree model includes a first parameter corresponding to a branching condition of an internal node for allocating the target patient to one leaf node of the decision tree, and a second parameter corresponding to a condition for converting information on the leaf node into an estimated value. In addition, the second parameter is a parameter that converts a representative feature representation vector of a patient population consisting of one or more other patients allocated to the leaf node into a representative value of the estimated value in the patient population. Therefore, the inference apparatus 30 according to the first embodiment can appropriately infer a treatment effect using the decision tree model.

Furthermore, according to the first embodiment, the causal inference model further includes a neural network that extracts the feature representation vector that does not depend on treatment allocation based on the attribute information for the other patient. Therefore, the inference apparatus 30 according to the first embodiment can obtain a bias-reduced estimation result.

Furthermore, according to the first embodiment, parameters of the causal inference model are updated as the knowledge information is updated. Therefore, the inference apparatus 30 according to the first embodiment can always perform inference using the appropriate causal inference model 342.

Furthermore, according to the first embodiment, the causal inference model further includes a neural network that estimates an individualized treatment effect based on a result of estimation by the decision tree model and the attribute information for the other patient. Therefore, the inference apparatus 30 according to the first embodiment can estimate an individualized treatment effect for each patient.

### Second Embodiment

In the first embodiment, the case in which a decision tree model is learned by applying a causal tree has been described. In a second embodiment, a case where a decision tree model is learned using knowledge distillation will be described. That is, in the second embodiment, an approach will be described in which a model capable of appropriately learning causality is learned first, and a decision tree is learned using a result thereof. Note that the inference apparatus 30 according to the second embodiment is different from that according to the first embodiment in the processing content of the learning function 353. Hereinafter, this point will be mainly described.

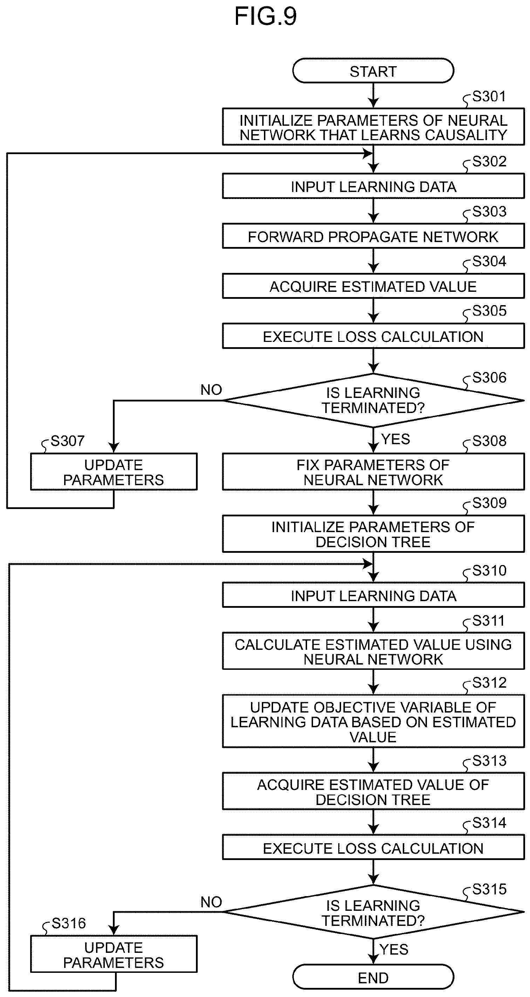

FIG. 9 is a flowchart illustrating an example of a procedure of a causal inference model construction process executed by the inference apparatus 30 according to the second embodiment. Here, FIG. 9 illustrates a case where a neural network that learns causality is learned in steps S301 to S308, and a decision tree model is learned in steps S309 to S316. Note that the processing of each step illustrated in FIG. 9 is realized by the processing circuitry 35 reading a program corresponding to the learning function 353 from the storage circuitry 34 and executing the program.

For example, as illustrated in FIG. 9, in the inference apparatus 30 according to the present embodiment, the learning function 353 initializes parameters of the neural network that learns causality (step S301), inputs learning data acquired by the acquisition function 352 (step S302), forward propagates the network (step S303), and acquires an estimated value (step S304).

Subsequently, the learning function 353 calculates a loss related to the acquired estimated value (step S305) and determines whether to terminate the learning (step S306). Here, when the learning is not terminated (step S306, No), the learning function 353 updates the parameters of the neural network (step S307) and inputs learning data again (step S302).

On the other hand, when the learning is terminated (step S306, Yes), the learning function 353 fixes the parameters of the neural network (step S308) and initializes the parameters of the decision tree model (step S309). Subsequently, the learning function 353 inputs learning data into the neural network with fixed parameters (step S310), and calculates an estimated value (step S311).

Subsequently, the learning function 353 updates an objective variable of the learning data input into the decision tree model based on the estimated value acquired from the neural network (step S312), and learns the decision tree model (step S313). Here, the learning function 353 performs loss calculation in all combinations of branching conditions (explanatory variables) of internal nodes of the decision tree model and thresholds (step S314), and determines whether to terminate the learning of the decision tree model (whether the loss satisfies the condition) (step S315).

Here, when the learning of the decision tree model is not terminated (step S315, No), the learning function 353 updates the parameters of the decision tree model (step S316) and inputs learning data again (step S310). On the other hand, when the learning of the decision tree model is terminated (step S315, Yes), the learning function 353 terminates the process.

Hereinafter, the causal inference model construction process executed by the inference apparatus 30 will be described in detail.

The learning function 353 according to the second embodiment optimizes the causal inference model 342 based on an estimated value obtained by a machine learning model that estimates a treatment effect for other patient.
Specifically, the learning function 353 learns treatment effects using a second causal inference model different from the causal inference model 342 in the learning of the neural network described in steps S301 to S308 of FIG. 9, and learns a decision tree model using estimated values obtained by the second causal inference model as teacher data in the learning of the decision tree model described in steps S309 to S316 of FIG. 9.

Here, as the second causal inference model, a model using "deep learning" (e.g., CounterFactual Regression (CFR) or disentangled representation learning (DRNet)) or a model using random forests (e.g., Causal Forest) can be used. Note that, for "CFR", the technology described in the literature "Uri Shalit, et al. "Estimating individual treatment effect: generalization bounds and algorithms" Proceedings of the 34 th International Conference on Machine Learning, Sydney, Australia, PMLR 70, 2017" can be used. In addition, for "DRNet", the technology described in the literature "Jiebin Chu, et al. "On learning disentangled representations for individual treatment effect estimation" Journal of Biomedical Informatics 124 (2021) 103940" can be used. In addition, for "Causal Forest", the technology described in the literature "Susan Athey, et al. "Estimating Treatment Effects with Causal Forests: An Application" Observational Studies 5 (2019) 36-51" can be used.

Here, in the learning of the second causal inference model, the knowledge information can be integrated by performing additional loss calculation. For example, the learning function 353 can integrate the knowledge information into the second causal inference model by optimizing the parameters of the second causal inference model using a method similar to the loss calculation using the knowledge information described in the first embodiment.

The learning function 353 acquires an estimated value by inputting learning data into the second causal inference model with the optimized parameters, and learns a decision tree model using the acquired estimated value as an objective variable. Here, since the teacher data (the estimated values obtained by the second causal inference model) used in the learning of the decision tree model has confounding bias reduced by the second causal inference model, the decision tree model itself does not need to address the confounding bias. Therefore, in the learning of the decision tree model here, a general decision tree model can be used.

For example, a model using a greedy method (e.g., CART), a model using a gradient descent method (e.g., GradTree), and a model using an evolutionary algorithm (e.g., "evtree" using a genetic algorithm) can be used as the decision tree model. Note that, for "evtree", the technology described in the literature "Thomas Grubinger, et al. "evtree: Evolutionary Learning of Globally Optimal Classification and Regression Trees in R" Working Papers in Economics and Statistics, No. 2011-20" can be used.

Here, in the present embodiment, when the decision tree model is learned, it is desirable to use a method capable of optimizing a branching condition (feature amount) used in branching each node and a threshold value, which are set as parameters, similarly to "GradTree". Such a method may be "CMA-ES" (covariance matrix adaptive evolution strategy) using an evolutionary algorithm. In addition, since the feature amount is a categorical variable, it is more desirable to use "CatCMA" corresponding to the categorical variable. Note that, for "CMA-ES", the technology described in the literature "Nikolaus Hansen. "The CMA Evolution Strategy: A Tutorial" arXiv:1604.00772v2" can be used. In addition, for "CatCMA", the technology described in the literature "Ryoki Hamano, et al. "Stochastic Optimization for Mixed Category Problems" arXiv:2405.09962v2" can be used.

Here, when the decision tree model is learned, the knowledge information can be integrated by fixing the tree structure converted from the knowledge information to a part of the decision tree model. For example, in the method using "CMA-ES", similarly to the method in "GradTree" described in the first embodiment, the learning function 353 can search for an optimal knowledge information integration location by treating a node to be fixed as a learning parameter.

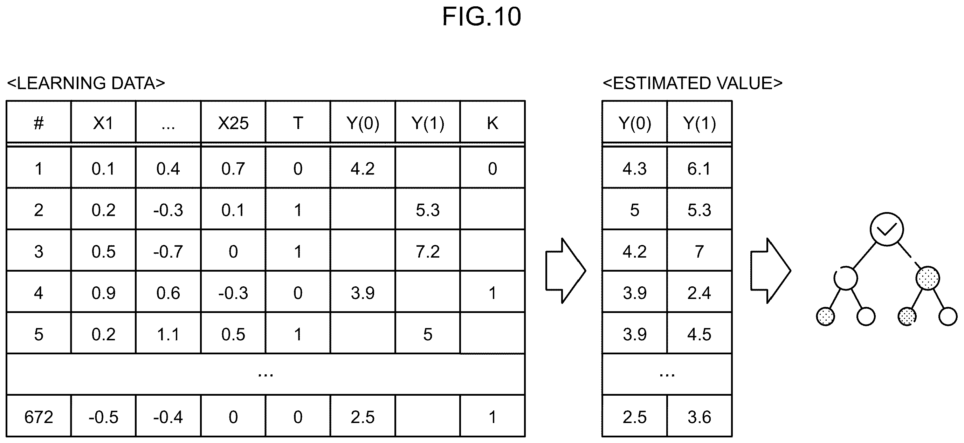

FIG. 10 is a diagram for explaining construction of the causal inference model 342 according to the second embodiment. For example, as illustrated in FIG. 10, the learning function 353 constructs a second causal inference model with parameters optimized by learning the second causal inference model using learning data, and inputs attribute information for each of the other patients into the constructed second causal inference model, thereby estimating treatment effects "Y(0)" and "Y(1)" for each of the other patients. The learning function 353 constructs the causal inference model 342 by learning a decision tree model using estimated values as teacher data. Note that the estimated values estimated by the second causal inference model may be "Y(0)" and "Y(1)", but may be a difference between "Y(0)" and "Y(1)".

The learning function 353 stores the causal inference model 342 constructed as described above in the storage circuitry 34. Similarly to the first embodiment, the estimation function 354 performs an inference process using the causal inference model 342 stored in the storage circuitry 34.

As described above, according to the second embodiment, the causal inference model is optimized based on an estimated value obtained by a machine learning model that estimates a treatment effect for other patient. Therefore, the inference apparatus 30 according to the second embodiment can accurately estimate a treatment effect for each patient using various methods.

### Other Embodiments

In the above-described embodiment, the causal inference model 342 is constructed by a single decision tree model. However, the embodiment is not limited thereto, and the causal inference model 342 may be an ensemble learning model using the decision tree model as a weak learner. For example, the causal inference model 342 may be constructed by a model combining a plurality of decision trees such as random forests. As a result, it is possible to obtain a model with higher accuracy than a causal inference model constructed from a single decision tree model.

Furthermore, in the above-described embodiment, each process is performed by an operation via the input interface 32 of the inference apparatus 30. However, the embodiment is not limited thereto, and the inference apparatus 30 may execute a process in response to an input from the terminal apparatus 20. That is, the inference apparatus 30 can be caused to perform each process by an operator's operation via the input interface of the terminal apparatus 20.

Note that, in the above-described embodiments, the processing units in the present specification are realized by the control function, the acquisition function, the learning function, and the estimation function of the processing circuitry, respectively, but the embodiments are not limited thereto. For example, the processing units in the present specification may be realized not only by the control function, the acquisition function, the learning function, and the estimation function described in the embodiments, but also by hardware alone, software alone, or a combination of hardware and software.

Furthermore, the term "processor" used in the description of the above-described embodiments refers to, for example, a central processing unit (CPU), a graphics processing unit (GPU), or a circuit such as an application specific integrated circuit (ASIC) or a programmable logic device (e.g., a simple programmable logic device (SPLD), a complex programmable logic device (CPLD), or a field programmable gate array (FPGA)). Here, instead of storing the program in the storage circuit, the program may be directly incorporated in the circuit of the processor. In this case, the processor realizes the function by reading and executing the program incorporated in the circuit. In addition, each processor of the present embodiment is not limited to being configured as a single circuit, and may instead be configured by combining a plurality of independent circuits into a single processor to realize its function.

Here, the program executed by the processor is provided by being incorporated in advance in a read only memory (ROM), a storage circuit, or the like. Note that this program may be provided as a computer program product by being recorded in a non-transitory computer-readable storage medium such as a compact disk (CD)-ROM, a flexible disk (FD), a CD-recordable (CD-R), or a digital versatile disk (DVD) as a file in an installable format or an executable format in this device. This program may also be provided or distributed as a computer program product by being stored on a computer connected to a network such as the Internet and downloaded via the network. For example, this program is configured by modules including the above-described processing functions. As actual hardware, the CPU reads a medical image processing program from the storage medium such as the ROM and executes the medical image processing program, whereby each module is loaded on the main storage device and generated on the main storage device.

In addition, in the above-described embodiments and modifications, the components of the apparatuses illustrated in the drawings are functionally conceptual, and do not necessarily need to be physically configured as illustrated in the drawings. That is, the specific form in which the apparatuses are distributed or integrated is not limited to that illustrated in the drawings, and all or some of the apparatuses can be functionally or physically distributed or integrated in any unit depending on various loads, usage conditions, and the like. Furthermore, all or some of the processing functions performed in each apparatus can be realized by a CPU and a program analyzed and executed by the CPU, or can be realized as hardware by wired logic.

In addition, among the processes described in the above-described embodiments and modifications, all or some of the processes described as being performed automatically can be manually performed, or all or some of the processes described as being performed manually can be automatically performed by a known method. In addition, the processing procedures, the control procedures, the specific names, and the information including various types of data and parameters illustrated in the above document and the drawings can be arbitrarily changed unless otherwise specified.

According to at least one of the embodiments described above, it is possible to accurately estimate a treatment effect for each patient.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the inventions as defined by the appended claims.

## Claims

1. An inference method comprising:
acquiring attribute information for a target patient to be subjected to inference; and
estimating a causal relationship between the attribute information for the target patient and a treatment effect for the target patient using a causal inference model including a decision tree model optimized based on knowledge information defining rules for determining a treatment to be applied to other patient and clinical information indicating relationship between attribute information for the other patient and other treatment effect observed after the treatment.

2. The inference method according to claim 1, wherein the knowledge information includes first knowledge information for specifying, for each treatment type, a condition of other patient for whom the treatment is recommended or a condition of other patient for whom the treatment is not recommended.

3. The inference method according to claims 1 or 2, wherein the knowledge information includes second knowledge information for specifying, for each attribute of other patient, a treatment type in which the attribute is advantageous or a treatment type in which the attribute is disadvantageous.

4. The inference method according to any one of claims 1 to 3, wherein at least some parameters of the decision tree model are determined so as to match the rules for determining the treatment in the knowledge information.

5. The inference method according to claim any one of claims 1 to 4, wherein the causal inference model is optimized by giving a penalty when a contradiction occurs in comparison between an estimation result and a treatment determination based on the knowledge information.

6. The inference method according to any one of claims 1 to 5, wherein the causal inference model is optimized based on an estimated value obtained by a machine learning model that estimates a treatment effect for other patient.

7. The inference method according to any one of claims 1 to 6, wherein the decision tree model includes a first parameter corresponding to a branching condition of an internal node for allocating the target patient to one leaf node of the decision tree, and a second parameter corresponding to a condition for converting information on the leaf node into an estimated value.

8. The inference method according to claim 7, wherein the second parameter is a parameter that converts a representative feature representation vector of a patient population consisting of one or more other patients allocated to a leaf node into a representative value of the estimated value in the patient population.

9. The inference method according to claim 8, wherein the causal inference model further includes a neural network that extracts the feature representation vector that does not depend on treatment allocation based on the attribute information for the other patient.

10. The inference method according to any one of claims 1 to 9, wherein the causal inference model is an ensemble learning model using the decision tree model as a weak learner.

11. The inference method according to any one of claims 1 to 10, wherein the parameters of the causal inference model are updated as the knowledge information is updated.

12. The inference method according to any one of claims 1 to 11, wherein the causal inference model further includes a neural network that estimates an individualized treatment effect based on a result of estimation by the decision tree model and the attribute information for the other patient.

13. An inference apparatus (30) comprising:
an acquisition unit (332) configured to acquire attribute information for a target patient to be subjected to inference; and
an estimation unit (334) configured to estimate a causal relationship between the attribute information for the target patient and a treatment effect for the target patient using a causal inference model including a decision tree model optimized based on knowledge information defining rules for determining a treatment to be applied to other patient and clinical information indicating relationship between attribute information for the other patient and other treatment effect observed after the treatment.

14. A storage medium that non-transiently stores a program for causing a computer to execute:
acquiring attribute information for a target patient to be subjected to inference; and
estimating a causal relationship between the attribute information for the target patient and a treatment effect for the target patient using a causal inference model including a decision tree model optimized based on knowledge information defining rules for determining a treatment to be applied to other patient and clinical information indicating relationship between attribute information for the other patient and other treatment effect observed after the treatment.
